**Europäisches Patentamt**

**European Patent Office**

**Office européen des brevets**

(11) Publication number: **0 271 563**

**B1**

# EUROPEAN PATENT SPECIFICATION

(45) Date of publication of patent specification: **29.08.90**

(51) Int. Cl.⁵: **B 01 J 21/16**

(21) Application number: **87904443.6**

(22) Date of filing: **11.06.87**

(86) International application number:
**PCT/US87/01442**

(87) International publication number:
**WO 88/00091 14.01.88 Gazette 88/02**

(54) **LAYERED SILICATES AND THEIR SYNTHESIS.**

(30) Priority: **27.06.86 US 879787**

(43) Date of publication of application:
**22.06.88 Bulletin 88/25**

(45) Publication of the grant of the patent:
**29.08.90 Bulletin 90/35**

(84) Designated Contracting States:
**BE DE FR GB IT NL SE**

(56) References cited:
**FR-A-2 394 324**
**US-A-4 510 257**
**US-A-4 579 832**
**US-A-4 637 991**

(73) Proprietor: **MOBIL OIL CORPORATION**
**150 East 42nd Street**
**New York New York 10017 (US)**

(72) Inventor: **JOHNSON, Ivy, Dawn**
**138 B. Willow Turn**
**Mount Laurel, NJ 08054 (US)**

(74) Representative: **Colmer, Stephen Gary**
**Patent Department c/o Mobil Services Company**
**Limited Mobil Court 3 Clements Inn**
**London WC2A 2EB (GB)**

Courier Press, Leamington Spa, England.

# EP 0 271 563 B1

**Description**

The present invention relates to layered silicates containing interlayer chalcogenides as well as a method for preparing the same.

Many layered materials are known which have three-dimensional structures which exhibit their strongest chemical bonding in only two dimensions. In such materials, the stronger chemical bonds are formed in two-dimensional planes and a three-dimensional solid is formed by stacking such planes on top of each other, the interactions between the planes being weaker than the chemical bonds holding an individual plane together. The weaker bonds generally arise from interlayer attractions such as Van der Waals forces, electrostatic interactions, and hydrogen bonding. In those situations where the layered structure has electronically neutral sheets interacting with each other solely through Van der Waals forces, a high degree of lubricity is manifested as the planes slide across each other without encountering the energy barriers that arise with strong interlayer bonding. Graphite is an example of such a material. The silicate layers of a number of clay materials are held together by electrostatic attraction provided by ions located between the layers. In addition, hydrogen bonding interactions can occur directly between complementary sites on adjacent layers, or can be provided by interlamellar bridging molecules.

Laminated materials such as clays may be modified to increase their surface area. In particular, the distance between the layers can be increased substantially by absorption of various swelling agents such as water, ethylene glycol, amines and, ketones, which enter the interlamellar space and push the layers apart. However, the interlamellar spaces of such layered materials tend to collapse when the molecules occupying the space are removed by, for example, exposing the clays to high temperatures. Accordingly, such layered materials having enhanced surface area are not suited for use in chemical processes involving even moderately severe conditions.

The extent of interlayer separation can be estimated by using standard techniques such as X-ray diffraction to determine the basal spacing, also known as "repeat distance" or "d-spacing". These values indicate the distance between, for example, the uppermost margin of one layer with the uppermost margin of its adjoining layer. If the layer thickness is known, the interlayer spacing can be determined by subtracting the layer thickness from the basal spacing.

Various approaches have been taken to provide layered materials of enhanced interlayer distance having thermal stability. Most techniques rely upon the introduction of an inorganic "pillaring" agent between the layers of a layered material. For example, U.S. Patent 4,216,188 discloses a clay which is crosslinked with metal hydroxide prepared from a highly dilute colloidal solution containing fully separated unit layers and a cross-linking agent comprising a colloidal metal hydroxide solution. However, this method requires a highly dilute forming solution of the clay (less than 1 g/l) in order to effect full layer separation prior to incorporation of the pillaring species, as well as positively charged species of cross linking agents.

U.S. Patent 4,248,739 describes stable pillared interlayered clay prepared from smectite clays reacted with cationic metal complexes of metals such as aluminum and zirconium. The resulting products exhibit high interlayer separation and thermal stability.

U.S. Patent 4,176,090 discloses a clay composition interlayered with polymeric cationic hydroxy metal complexes of metals such as aluminum, zirconium and titanium. Interlayer distances of up to 16 Angstrom are claimed although only distances restricted to about 9 Angstrom are exemplified for calcined samples. These distances are essentially unvariable and related to the specific size of the hydroxy metal complex.

Silicon-containing materials are believed to be a highly desirable species of pillaring agents owing to their high thermal stability characteristics. U.S. Patent 4,367,163 describes a clay intercalated with silica prepared by impregnating a clay substrate with a silicon-containing reactant such as an ionic silicon complex, e.g., silicon acetylacetonate, or a neutral species such as $SiCl_4$. The clay may be swelled prior to or during silicon impregnation with a suitable polar solvent such as methylene chloride, acetone, benzaldehyde, tri- or tetraalkylammonium ions, or dimethylsulfoxide. This method, however, appears to provide only a monolayer of intercalated silica resulting in a product of small spacing between layers, about 0.2—0.3 nm as determined by X-ray diffraction.

In a first aspect, the present invention resides in a layered product comprising a non-swellable, layered silicate containing non-silicon framework atoms and pillars of an oxide of at least one element selected from Groups IB, IIB, IIIA, IIIB, IVA, IVB, VA, VB, VIA, VIIA, VIIIA of the Periodic Table of the Elements (Fisher Scientific Co. Cat. No. 5-702-10, 1978) separating the silicate layers. Preferably, such materials are thermally stable, i.e., capable of withstanding calcination at a temperature of about 450°C for at least 2 hours without significant reduction (e.g., not greater than 10 or 20%) in the spacing between the silicate layers. Preferably said pillars are formed of a polymeric oxide and the product has a d-spacing of at least 2.0 nm.

For present purposes, polymeric oxides are considered to include oxides of two or more repeating units preferably three or more repeating units, say four or more or even five or more repeating units. The extent of polymerization of the interspathic polymeric oxide is believed to affect the ultimate interlayer separation of the layered product.

It is also to be understood that the term "layered" silicate is used herein in its commonly accepted sense to refer to a material which comprises a plurality of separate silicate layers which are capable of being physically displaced away from one another such that the spacing between adjacent layers is

2

increased. Such displacement can be measured by X-ray diffraction techniques and/or by density measurements.

In another aspect, the present invention relates to a method for preparing a layered product having adjacent layers separated by pillars of an oxide of at least one element selected from Groups IB, IIB, IIIA, IIIB, IVA, IVB, VA, VB, VIA, VIIA, and VIIIA of the Periodic Table which method comprises the steps of starting with a non-swellable layered silicate which contains non-silicon framework atoms and which has anionic sites associated therewith, physically separating the layers of the silicate by introducing an organic cationic species between the layers of the anionic sites, introducing between the separated silicate layers a compound capable of conversion to an oxide and converting said compound to the oxide to produce pillars of the oxide separating adjacent layers of the silicate.

Preferably, the layered silicate is a layered silicic acid, e.g., a high silica alkali silicate such as synthetic magadiite, synthetic makatite or synthetic kenyaite which is composed of only tetrahedral sheets condensed on each other without interposed octahedral sheets and which is synthesized by co-crystallizing in the presence of one or more non-silicon elements selected from Groups IB, IIA, IIB, IIIB, IVA, IVB, VA, VB, VIA, VIIA and VIIIA of the Periodic Table, most preferably from the group consisting of Al, Zr, B, Cr, Fe, Ga, In and Ni. Preferably the pillars are formed of polymeric silica or a mixture of polymeric silica and polymeric alumina.

The method of the invention is particularly useful in that it permits the preparation of layered silicates of relatively high interplanar distance (d-spacing), e.g., greater than about 10 nm and preferably greater than 2.0 nm up to or even exceeding 3.0 nm. These materials are capable of being exposed to severe conditions such as those encountered in calcining, e.g., at temperatures of about 450°C for about two or more hours, e.g., four hours, in nitrogen or air, without significant decrease, say, e.g., less than about 10%, in interlayer distance. Furthermore, such layered silicates can be prepared without the severe dilution often necessary to introduce the interspathic material in prior art techniques of interlayering. Finally, the size of interspathic oxide pillars contained within the final product can be greatly varied because the oxide precursor species can be introduced in an electrically neutral form such that the amount of interspathic material incorporated within the layered silicate is not dependent upon the charge density of the original layered silicate. Charge density should be taken into consideration in determining the suitability of the cationic species introduced between the layers in the procedure used to prop open the layers prior to pillaring.

The layered silicate employed herein is "non-swellable" which is intended to distinguish from conventional clays which contain octahedrally coordinated metal oxide sheets bonded to tetrahedrally coordinated silica sheets and which undergo substantial swelling, sometimes by an essentially unbounded amount, when contacted with water. As used herein in relation to a layered silicate material, the term "non-swellable" is defined as meaning a layered silicate which, when contacted with at least 10 grams of water per gram of the layered silicate at 23°C for 24 hours, exhibits an increase in d-spacing no greater than 0.5 nm as compared with the material before treatment. Included among these materials are magadiite, natrosilite, kenyaite, makatite, nekoite, kanemite, okenite, dehayelite, macdonaldite and rhodesite which, unlike swellable clays, lack octahedral sheets, i.e., sheets composed of atoms which are octahedrally coordinated with oxygen atoms. Without stable intercalated pillars, these materials tend to have collapsed layers at elevated temperatures, low porosity and low surface area. In some cases it has been found preferable that these layered silicates be treated by contacting with one or more polar organic solvents or water prior to or during exchange with the source of organic cation. The polar organic solvent used should exhibit electric dipole moments in the gas phase of at least 3.0 Debyes (D), preferably at least 3.5 Debyes, most preferably at least about 3.8D. Examples of suitable organic solvents are dimethylsulfoxide (DMSO) and dimethylformamide (DMF). A table of selected organic compounds and their electric dipole moments can be found in CRC Handbook of Chemistry and Physics, 61st Edition, 1980—1981 at pages E-64 to E-66.

The layered silicates employed in the present invention are preferably high silica alkali silicates whose layers lack octahedral sheets and which are prepared hydrothermally from aqueous reaction mixture containing silica and caustic at relatively moderate temperatures and pressures. The layered silicates contain tetracoordinate framework atoms other than Si in the layers and which can be prepared by co-crystallizing in the presence of non-silicon tetravalent elements, e.g., those selected from the group consisting of Al, B, Co, Cr, Fe, Ga, In, Ni, Zr as well as any other such elements which are catalytically useful when incorporated in the silicate sturcture. Alternatively, non-silicon framework elements already in a layered silicate may be substituted by a different tetracoordinate element. For example, kenyaite containing boron in its framework when treated with aluminum nitrate results in a kenyaite which contains aluminum in its framework. Both co-crystallized and substituted layered high silica alkali silicates may be treated by the present invention to provide layered materials containing interspathic oxide pillars.

One suitable layered silicate is synthetic substituted magadiite. Synthetic magadiite is readily produced hydrothermally from a reaction mixture containing inexpensive sources of silica and caustic. Tetracoordinate elements X other than silicon, e.g., Al, B, Co, Cr, Fe, Ga, In, Ni, Zr, preferably Al or Fe, may be added to the reaction mixture as may a suitable organic directing agent R. The reaction mixture for such synthetic magadiite-type materials can be described in molar ratios as follows:

$SiO_2/X_2O_3 = 10$ to infinity where X can be Al, B, Co, Cr, Fe, Ga, and/or Ni or other catalytically useful metal

$M^+OH^-/SiO_2 = 0$ to 0.6, (preferably 0.1—0.6) M=any alkali metal

$H_2O/SiO_2 = 8$—500

$R/SiO_2 = 0$—0.4

where R can be an organic such as benzyltriethylammonium chloride, benzyltrimethylammonium chloride, dibenzyldimethylammonium chloride, N,N'-dimethylpiperazine, triethylamine, or other quaternary compounds or heterocyclic amines.

The reaction mixture is maintained at a temperature of 100 to 200°C for anywhere from 1 to 150 days in order to form a product having the following composition:

$\%N = 0$—3, e.g., 0 to 0.3

$SiO_2/X_2O_3 = 10$ to infinity where X is in the tetrahedral or octahedral position

$M_2O/SiO_2 = 0$ to 0.5, e.g., 0.05—0.1.

The synthetic layered silicate material thus prepared is of low surface area. Introduction of interspathic polymeric oxides according to the method of the present invention can increase the surface area of the material. Generally, the synthetic magadiite-type material is acidified by any suitable means, e.g., treatment with aqueous 0.1 N HCl, before being treated with a "propping" agent, alone or combined with a suitable polar solvent as discussed above.

Another suitable layered silicate is a synthetic kenyaite-type material. Kenyaite, a layered silicic acid which is known to exist in nature as a sodium salt $Na_2Si_{22}O_{45}H_2O$ can be prepared in the potassium form $K_2Si_{22}O_{45}10H_2O$ in the laboratory from a reaction mixture containing inexpensive sources of silica and caustic, preferably KOH. Tetracoordinate elements other than silicon, e.g., those selected from the group consisting of Al, B, Co, Cr, Fe, Ga, In, Ni, Zr and other catalytically useful metals, may be added to the reaction mixture to produce synthetic kenyaite-type layered silicates. $Al(NO_3)_3 \cdot 9H_2O$ and aluminum-tri-sec-butoxide are suitable reagents for the introduction of non-silicon tetracoordinate elements in the kenyaite framework. Co-crystallizing with B, Al, Ga, Fe and/or Zr is particularly preferred. The reaction mixture may also be seeded with kenyaite.

The layered silicate starting material used in the method of the invention contains anionic sites having interlayer cations associated therewith. Such cations may include hydrogen ion, hydronium ion aluminum ion and alkali metal cation. The starting material is treated with a "propping" agent comprising a source of organic cation such as an organoammonium cation in order to effect an exchange of or addition to the interlayer cations in the starting material thereby resulting in the layers of the starting material being propped apart. The source of organic cation in those instances where the interlayer cations include hydrogen or hydronium ions may include a neutral compound such as organic amine which is converted to a cationic analogue during the "propping" treatment. In some instances, it may be desirable to remove excess propping agent which is not electrostatically bound within the layered starting material in order to permit the subsequent addition of greater amounts of polymeric oxide precursor. Such removal may be effected by washing out the propping agent with a suitable solvent.

The foregoing treatment results in the formation of a layered silicate of enhanced interlayer separation depending upon the size of the organic cation introduced. In one embodiment, a series of organic cation exchanges is carried out. For example, an organic cation may be exchanged with an organic cation of greater size, thus increasing the interlayer separation in a step-wise fashion. Preferably, contact of the layered oxide with the propping agent is conducted in aqueous medium so that water is trapped between the layers of the "propped" silicate.

After the ion exchange, the organic-"propped" species is treated with a compound capable of conversion, preferably by hydrolysis, to pillars of a polymeric oxide. Where the treatment involves hydrolysis, this may be carried out using water already present in the organic-"propped" layered silicate. In this case, the extent of hydrolysis may be modified by varying the extent to which the organic-"propped" species is dried prior to addition of the polymeric oxide precursor.

It is preferred that the organic cation deposited between the layers be capable of being removed from the layered silicate without substantial disturbance or removal of the interspathic polymeric oxide or its precursor. For example, organic cations such as n-octylammonium may be removed by exposure to elevated temperatures, e.g., calcination in nitrogen or air, or by chemical oxidation, preferably after the interspathic polymeric oxide precursor has been converted to the polymeric oxide.

The products of the present invention, especially when calcined, exhibit high surface area, e.g., greater than 200, 400 or even 600 m²/g, and thermal and hydrothermal stability making them highly useful as catalysts or catalytic supports for hydrocarbon conversion processes, for example, cracking and hydrocracking.

As previously stated, the layer silicate starting material is treated with an organic compound capable of forming cationic species such as organophosphonium or organoammonium ion, before adding the polymeric oxide source. Insertion of the organic cation between the adjoining layers serves to physically separate the layers in such a way as to make the layered silicate receptive to the interlayer addition of an electrically neutral, hydrolyzable, polymeric oxide precursor. In particular, alkylammonium cations have been found useful in the present invention. Thus $C_3$ and larger alkylammonium, e.g., n-octylammonium, cations are readily incorporated within the interlayer spaces of the layered silicate, serving to prop open the layers in such a way as to allow incorporation of the polymeric oxide precursor. The extent of the interlayer spacing can be controlled by the ize of the organoammonium ion employed so that use of the n-propylammonium cation can achieve an interlayer spacing of 0.2 to 0.5 nm whereas to achieve an interlayer opening of 1.0 to 2.0 nm an n-octylammonium cation or a cation of equivalent length is required. Indeed, the size and shape of the organic cation can affect whether or not it can be incorporated within the layered silicate structure at all. For example, bulky cations such as tetrapropylammonium are generally undesirable for use in the present method while n-alkyl ammonium cations such as those derived from primary n-alkyl amines and $R_3R'N^+$ cations, where R is methyl or ethyl and R' is an n-alkyl group with at least 5 carbon atoms, are preferred. The organic ammonium cations separating the silicate layers may also be formed in situ by reaction of a neutral amine species with interlayer hydrogen or hydronium cations of the layered starting material. Alternatively, where the interlayer cations of the layered starting material are alkali metal cations, the organic ammonium cation may be formed by initially combining an amine and an aqueous acid solution, such as hydrochloric acid, and then treating the layered silicate with the resulting aqueous organoammonium ion solution. In either case, the treatment is preferably conducted in aqueous media so that water is then available to hydrolyze the electrically neutral, hydrolyzable polymeric oxide precursor subsequently introduced into the "propped" product.

The interspathic polymeric oxide pillars formed between the layers of the silicate starting material may be an oxide of zirconium or titanium or more preferably of an element selected from Group IVB of the Periodic Table (Fischer Scientific Company Cat. No. 5-702-10, 1978), other than carbon, i.e., silicon, germanium, tin and lead. Other such elements may include those of Group VA, e.g., V, Nb, and Ta, those of Group IIA, e.g., Mg or those of Group IIIB, e.g., B. Most preferably, the pillars include polymeric silica. In addition, the polymeric oxide pillars may include an element which provides catalytically active acid sites in the pillars, preferably aluminum.

The polymeric oxide pillars are formed from a precursor material which is preferably introduced between the layers of the organic "propped" species as a cationic, or more preferably, electrically neutral, hydrolyzable compound of the desired elements, e.g., those of Group IVB. The precursor material is preferably an organometallic compound which is a liquid under ambient conditions. In particular, hydrolyzable compounds, e.g., alkoxides, of the desired elements of the pillars are utilized as the precursors. Suitable polymeric silica precursor materials include tetraalkylsilicates, e.g., tetrapropylorthosilicate, tetramethylorthosilicate and, most preferably, tetraethylorthosilicate. Where the pillars are also required to include polymeric alumina, a hydrolyzable aluminum compound can be contacted with the organic "propped" species before, after or simultaneously with the contacting of the layered silicate with the silicon compound. Preferably, the hydrolyzable aluminum compound employed is an aluminum alkoxide, e.g., aluminum isopropoxide. If the pillars are to include polymeric titania, a hydrolyzable titanium compound such as titanium alkoxide, e.g., titanium isopropoxide, may be used.

In addition, the polymeric oxide precursor may contain zeolite precursors such that exposure to conversion conditions results in the formation of interspathic zeolite material as at least some of the polymeric oxide pillars. In this case, the source of organic cation propping agent may act as a zeolite synthesis directing agent. U.S. Patent No. 4,151,189 discloses reagents and conditions suitable in forming the zeolite component of this embodiment. The patent discloses oxides of aluminum, silicon and alkali metal suitable for zeolite synthesis as well as zeolite synthesis directing agents such as sources of organic nitrogen cation, like $C_4$—$C_{10}$ n-alkylamines. Suitable sources of alumina include sodium aluminate, aluminum sulfate and alumina while suitable sources of alkali metal include alkali metal hydroxide such as sodium hydroxide. Suitable reaction conditions include heating the layered material containing the zeolite precursors to a temperature of from 99°C to 260°C for a period from 6 hours to 60 days, preferably 149°C to 232°C for a period from 12 hours to 8 days. The resulting layered material comprising interspathic zeolite may be subsequently treated by ion exchange and/or calcining.

After hydrolysis to produce the polymeric oxide pillars and calcination to remove the organic propping agent, the final pillared product may contain residual exchangeable cations. Such residual cations in the layered material can be ion exchanged by known methods with other cationic species to provided or alter the catalytic activity of the pillared product. Suitable replacement cations include cesium, cerium, cobalt, nickel, copper, zinc, manganese, platinum, lanthanum, aluminum, ammonium, hydronium and mixtures thereof.

When used as a catalyst, it may be desirable to incorporate the pillared product of the invention with another material, i.e. a matrix, resistant to the temperatures and other conditions employed in organic conversion processes. Such materials include active and inactive materials and synthetic or naturally occurring zeolites as well as inorganic materials such as clays, silica and/or metal oxides. The latter may be either naturally occurring or in the form of gelatinous precipitates or gels including mixtures of silica and metal oxides. Use of a matrix in conjunction with the pillared product, i.e. combined therewith, which is active, tends to improve the conversion and/or selectivity of the catalyst in certain organic conversion processes. Inactive materials suitably serve as diluents to control the amount of conversion in a given process so that products can be obtained economically and orderly without employing other means for controlling the rate of reaction. These materials may be incorporated into naturally occurring clays, e.g. bentonite and kaolin, to improve crush strength of the catalyst under commercial operating conditions. Said materials, i.e. clays, oxides, etc., function as binders for the catalyst.

It is desirable to provide a catalyst having good crush strength because in commercial use it is desirable to prevent the catalyst from breaking down into powder-like materials. These clay binders have been employed normally only for the purpose of improving the crush strength of the catalyst.

Naturally occurring clays which can be composited with the pillared product include montmorillonite and kaolin families which include the subbentonites, and the kaolines commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Matrix materials useful for compositing with the pillared product also include inorganic oxides, notably alumina or silica.

In addition to the foregoing materials, the pillared product of the invention can be composited with a porous matrix material such as aluminum phosphate, silica-alumina, silica-magnesia, silica-zirconia, silic-thoria, silica-beryllia, silica-titania as well as ternary compositions such as silica-alumina-thoria, silica-alumina-zirconia, silica-alumina-magnesia and silica-magnesia-zirconia. The relative proportions of finely divided pillared product and inorganic oxide gel matrix vary widely, with the content of the pillared product ranging from 1 to 90 percent by weight and more usually, particularly when the composite is prepared in the form of beads or extrudates, in the range of 2 to 80 weight percent of the composite.

The invention will now be more particularly described with reference to the following examples. In these examples, adsorption data were determined as follows: A weighed sample was contacted with the desired pure adsorbate vapor at a pressure less than the vapor-liquid equilibrium pressure of the adsorbate at room temperature. Adsorption was complete when a constant pressure in the adsorption chamber was reached (overnight for water, 3 hours for hydrocarbons); e.g., 12 mm of mercury for water and 40 mm for n-hexane and cyclohexane. Samples were then removed and weighed. The increase in weight was calculated as the adsorption capacity of the samples. Nitrogen BET surface areas were reported in $m^2/g$. X-ray diffraction data was obtained by standard techniques using K-alpha doublet of copper radiation. When alpha value is examined, it is noted that the alpha value is an approximate indication of the catalytic cracking activity of the test catalyst compared to a standard catalyst and gives the relative rate constant (rate of normal hexane conversion per volume of catalyst per unit time) compared to the activity of the highly active silica alumina cracking catalyst taken as an alpha of 1 (Rate Constant=0.16 $sec^{-1}$). The alpha test is described in U.S. Patent 3,354,078 and in *The Journal of Catalysis*, Vol. IV, pp. 522—529 (August 1965).

Examples 1—5

Preparation of synthetic magadiite co-crystallized with aluminum and/or Fe and intercalated with silica

Example 1

24.5 g of Dibenzyldimethylammonium chloride were added to a solution containing 0.63 g sodium aluminate (43.3% $Al_2O_3$, 32.2% $Na_2O$, 25.6% $H_2O$), 4.0 g NaOH and 30.0 g $H_2O$. The mixture was added to 134.0 g colloidal $SiO_2$ (30%) and thoroughly mixed. The mixture was heated at 140°C in a static reactor for 21 days. X-ray analysis indicated a synthetic magadiite material containing trace amounts of mordenite.

Example 2

2.49 g of $FeNH_4(SO_4)_2 \cdot 12H_2O$ were dissolved in 11.0 g of $H_2O$. This mixture was added to a solution of 5.23 g NaOH dissolved in 11.0 g $H_2O$. The resulting mixture was added to 72.6 g of a 40% $SiO_2$ colloidal silica solution and thoroughly mixed and then crystallized in a static reactor at 150°C for 21 days. X-ray analysis indicated a synthetic magadiite material.

Example 3

A solution containing 5.16 g of $FeNH_4(SO_4)_2$ in 10.0 g $H_2O$ was mixed with a second solution containing 6.86 g dibenzyldimethylammonium chloride, 6.86 g NaOH and 25.0 g $H_2O$. The mixture was added to 136 g of 30% colloidal silica solution and thoroughly mixed. The reaction mixture was crystallized at 150°C for 23 days. X-ray analysis indicated a synthetic magadiite material containing trace amounts of zeolite beta.

## Example 4

The following solutions were prepared:

| | | | |
|---|---|---|---|
| A. | NaAlO$_2$, | | 1.5 g |
| | Benzyltriethylammonium chloride | | 64.5 g |
| | H$_2$O | | 180.0 g |
| B. | NaCl | | 22.2 g |
| | H$_2$O | | 345.0 g |
| C. | Q-Brand sodium silicate (28.8% SiO$_2$, 8.9% Na$_2$O) | | 156.0 g |
| | H$_2$O | | 510.0 g |
| D. | HCl (conc.) | | 8.5 g |
| | H$_2$O | | 200.0 g |

A was added to B.
AB was added to C.
D was added to ABC and mixed well.

The reaction mixture was crystallized in a stirred reactor at 150°C for 7 days. X-ray analysis indicated a synthetic magadiite material.

## Example 5

The following reagents were prepared:

| | | |
|---|---|---|
| A. | NaAlO$_2$, g | 0.96 g |
| | NaOH | 2.8 g |
| | H$_2$O | 13.60 g |
| B. | Benzyltributylammonium chloride | 22.3 g |
| C. | Colloidal silica (30%). | |

B was dissolved in A and mixed with C. The mixture was crystallized in a static reactor at 100°C for 215 days. X-ray analysis indicated a synthetic magadiite material.

The compositions and properties of the layered materials of Example 1 to are set out below in Table 1.

### TABLE 1
#### Compositions and properties of as-synthesized layered silicates

| Example | 1 | 2 | 3 | 4 | 5 |
|---|---|---|---|---|---|
| Composition, wt % | | | | | |
| SiO$_2$ | 84.7 | 78.0 | 80.0 | 79.6 | 79.6 |
| Al$_2$O$_3$ | 0.73 | 0.18 | 0.05 | 2.1 | 2.0 |
| Fe | — | 0.83 | 1.1 | — | — |
| Na | 3.6 | 4.2 | 3.4 | 3.7 | 3.9 |
| N | 0.26 | — | 0.25 | 0.07 | 0.07 |
| Ash | 88.7 | 85.6 | 87.0 | 89.5 | 88.1 |
| SiO$_2$/Al$_2$O$_3$ | 200 | 750 | 2600 | 64.4 | 67.7 |
| Surface area, m$^2$/g | 56 | 5 | 95 | | |
| Sorption properties | | | | | |
| Cyclohexane, wt % | 2.1 | 0.5 | 3.9 | | |
| n-Hexane | 2.2 | — | | | |
| H$_2$O | 7.7 | 2.9 | 10.4 | | |

These materials were then intercalated by the following procedure:

Twenty-five parts by weight of each solid sample were added to one hundred parts of H$_2$O. The pH of each slurry was gradually adjusted to 2 by adding 0.1 N HCl solution at room temperature and kept at a pH of 2 for 24 hours. Each solid was filtered and water washed and dried. To each dried sample, a mixture of 20 parts dimethylsulfoxide and 10 parts of n-octylamine was added and reacted for about 24 hours. Each mixture was filtered and dried on the filter for 3 hours. Each sample was then added to 100 parts of tetraethylorthosilicate at room temperature. The samples were filtered after a contact time of 24 hours. After drying, the samples were calcined at 37.8°C (1000°F) in air for 3 hours. Analysis of each product indicated a crystalline porous material. The compositions and properties of the resulting products are set out in Table 2 below.

TABLE 2
Properties of pillared silicates

| Base material | Ex. 1 | Ex. 2 | Ex. 3 | Ex. 4 | Ex. 5 |
|---|---|---|---|---|---|
| Surface area, m²/g | 486 | 598 | 583 | 360 | 634 |
| Sorption capacity, % | | | | | |
| $H_2O$ | 17.9 | 22.6 | 23.1 | — | — |
| $CyC_6$ | 14.8 | 19.8 | 19.1 | 10.9 | — |
| N—$C_6$ | 12.6 | 18.3 | 19.2 | — | — |
| Basal d-spacing, A | 24.0 | 24.5 | 25.9 | 25.2 17.7 | 30.5 |
| | 48 (mordenite contaminant) | 0.5 | 2.0 | 1.0 (zeolite beta contaminant) | 0.6 |

**Example 6**

Preparation of synthetic magadiite co-crystallized with aluminum and intercalated with polymeric silica

16.7 Grams of dibnezyldimethylammonium chloride were dissolved in a solution containing 0.42 grams of sodium aluminate (43.3% $Al_2O_3$, 32.2% $Na_2O$, 25.6% $H_2O$), 4.0 g NaOH and 20.0 g $H_2O$. The mixture was then added to 90.0 g of colloidal silica (30%) and thoroughly mixed. The mixture was heated at 140°C for 21 days in a static reactor at 118°C.

The resulting material was identified as a co-crystallized synthetic magadiite and had the following composition:

| | |
|---|---|
| N, wt % | 0.28 |
| Na | 3.6 |
| $Al_2O_3$ | 0.75 |
| $SiO_2$ | 81.0 |
| Ash | 83.49 |
| $SiO_2/Al_2O_3$ molar ratio | 184 |

A portion of the material after being calcined for 16 hours at 540°C had the following properties:

Cyclohexane adsorption, 40 torr=1.3 g/100 g.
Surface area of calcined material=29 m²/g.

15 Grams of the uncalcined dried (118°C) product were contacted with 20 cc of 10% $NH_4Cl$ solution per gram of material at 85°C with stirring for five one hour contacts. The Na level was reduced to below 0.01%. The washed product was dried at 118°C, calcined for 3 hours at 540°C in air and had an alpha value of 28.

Five grams of the dried, uncalcined material was added to 40 ml of $H_2O$. 0.1N HCl was added dropwise to obtain and maintain the solution at 2 pH for 24 hours. The mixture was filtered, water-washed and dried in air for 24 hours. The dried sample was then treated with a solution mixture of 10 g of dimethylsulfoxide and 5 g of octylamine for 24 hours at ambient temperature. The sample was again filtered and air dried on the filter for 3 hours. The X-ray diffraction pattern of the dried sample had a main low angle peak at 3.4° 2 theta indicating a basal spacing of 2.59 nm.

The resulting material was added to 20 g of tetraethylorthosilicate for 24 hours. The sample was filtered, dried at 110°C for 3 hours and calcined at 540°C for 3 hours in air. The X-ray diffraction pattern of the calcined sample showed a 3.8° (2 theta) low angle peak indicating a basal spacing of 2.32 nm. The adsorptive properties were: surface area 415 m²/g, $H_2O$ adsorption at 12 torr, 14.2%, cyclohexane at 40 torr, 10.9% and n-hexane at 40 torr, 9.4%. Two grams of the calcined example were slugged and sized to 14 to 25 mesh (1.2—0.8 nm) material. The sample was then tested for cracking catalytic activity by the alpha test. An alpha value of 14 was obtained indicating a very catalytically active material.

8

## Example 7

Preparation of synthetic kenyaite co-crystallized with aluminum and intercalated with polymeric silica

Aluminum-tri-sec-butoxide (14.1 g) was dissolved in $H_2O$ (150 g) and stirred overnight (approximately 18 hrs). The solution was slowly added to colloidal silica gel (172 g, Ludox LS, 30% $SiO_2$).

To this mixture KOH (10 g), which had been dissolved in $H_2O$ (50 g), was slowly added. 5 g of a pure silica kenyaite was added to seed the reaction. The reaction mixture was aged in a steambox for 2 hrs and the resulting gel dispersed in a blender then charged to an autoclave. The mixture was crystallized at 150°C for 120 hrs while stirring under autogenous pressure. The product was filtered, washed with distilled $H_2O$, and air-dried for characterization. X-ray diffraction indicated a kenyaite product. The surface area was 34 $m^2/g$ and chemical analysis indicated a $SiO_2/Al_2O_3$ of about 25. 40 Grams of the resulting material were suspended in 400 ml of $H_2O$. To this slurry, 1N HCl was added until the pH equalled 2. The stirring of the suspension was continued for 24 hrs whereafter the suspension was filtered, washed, and air-dried. The product (7 g) was resuspended in 20 g of $H_2O$ and octylamine (20 g) was added to the resulting suspension. This was stirred approximately 18 hrs, heated for approximately 1 hr, filtered, washed with warm $H_2O$, air-dried for 24 hrs and then dried in a vacuum oven at 71°C (160°F) for 3 hrs. The dried product was slurried in tetraethylorthosilciate (TEOS) (35 g) in a closed polypropylene jar for 72 hrs. The material was filtered, air-dried, and calcined at 500°C for 4 hrs. The final product had an X-ray low-angle diffraction line at 38.4 A.

## Example 8

Preparation of synthetic kenyaite co-crystallized with aluminum and intercalated with polymeric silica

$Al(NO_3)_3 . 9H_2O$ (6.42 g) was dissolved in $H_2O$ (100 g) and slowly added to colloidal silica (200 g, Ludox LS, 30% $SiO_2$). To this mixture KOH (12 g) which had been dissolved in $H_2O$ (100 g), was added. The mixture was aged in a steambox for 1/2 hour and the resulting gel was dispersed in a blender. The gel was charged to an autoclave and crystallized at 150°C for 120 hrs while stirring under autogenous pressure. The product was filtered, washed with distilled $H_2O$, and air-dried. X-ray diffraction pattern indicated a kenyaite product, while nmr and chemical analysis confirmed that substantially all the aluminum was tetrahedrally coordinated in the silicate lattice 40 grams of the resulting product which had a $SiO_2/Al_2O_3$ molar ratio of about 100 were suspended in 400 mls of $H_2O$. To this slurry, 1N HCl was added until the pH was about 2. The suspension continued to stir for 24 hrs, was filtered, washed, and air-dried. The product (7 g) was resuspended in 20 g of $H_2O$ and octylamine (20 g) was added to the resulting suspension. This was stirred approximately 18 hrs, heated for approximately 1 hr, filtered, washed with warm $H_2O$, air-dried for 24 hrs, then dried in a vacuum oven at 71°C (160°F) for 3 hrs. The dried product was slurried in tetraethylorthosilicate (TEOS) (35 g) in a closed polypropylene jar for 72 hrs. The material was filtered, air-dried, and calcined at 500°C for 4 hrs. The final product had an X-ray low-angle diffraction line at 3.84 nm.

## Examples 9—13

$H_3BO_3$ (21.3 g) was dissolved in $H_2O$ (300 g) and added to colloidal silica (515 g Ludox LS—30% $SiO_2$). To this mixture, a KOH solution (30 g, pellets—85% KOH in 300 g $H_2O$) was added. The gel was dispersed in a blender to assure adequate mixing. Due to the large volume of reaction mixture, it was divided in half and charged to two 600 ml autoclaves. They were both crystallized at 150°C for 168 hrs while stirring under autogenous pressure. The products were filtered, washed with $H_2O$, and air-dried for subsequent characterization. The major X-ray diffraction lines were at 2.0 nm, assigned as the basal spacing, and 0.34 nm which was assigned to an in-plane reflection. Portions of the layered silicate (10 g each) were slurried in aqueous solutions of metal (M) salt solutions, 0.05 molar each $Fe(NO_3)_3$, $Cr(NO_3)_3$, $TCl_3$, and $La(NO_3)_3$. They were heated to 60°C for 2 hrs without stirring. They were then filtered, washed with warm $H_2O$, and air-dried.

The resultant [M] kenyaites were pillared as in the previous examples to give crystalline molecular sieves. The X-ray diffraction pattern of these materials showed broad lines corresponding to basal spacings of about 2.3 nm which translates into an interlayer distance of 0.6 nm. The data on these materials is included in Table 3.

## Example 14

An Al-containing kenyaite was prepared in an analogous manner similar to that described in Examples 9—13 above except that 40 g of the air-dried product was stirred in 200 mls of 1M $Al(NO_3)_3$ solution at 80°C for 24 hours. This procedure was repeated, then the product was filtered, washed and air-dried. The composition of the product is included in Table 3. This material was characterized via Al nmr which showed that there was both tetrahedral (i.e., zeolitic) and octahedral aluminum present in the material. The signal for the octahedral aluminum was very narrow and indicated that the Al was hydrated $Al^{3+}(H_2O)_6$ located in the innerlayer. The product was pillared as in the previous examples to give a crystalline molecular sieve with a basal spacing of 3.2 nm. The composition and properties of the layered phase and pillared product are given in Table 3. The hexane cracking activity of this material, $\alpha$, is 1.

TABLE 3

| | Ex. 9 M=B[a] | Ex. 10 Fe | Ex. 11 Cr | Ex. 12 Ti | Ex. 13 La | Ex. 14 Al |
|---|---|---|---|---|---|---|
| Layered phases: | | | | | | |
| $SiO_2/M$ | 96 | 35 | 270 | 72 | 43 | 40 |
| $SiO_2/KOH$ | 17 | 780 | 1000 | 360 | 255 | ∞ |
| Surface area (m²/g) | NA | 177 | 139 | 172 | 142 | 151 |
| Pillared products | | | | | | |
| $SiO_2/M$ | NA | 50 | 95 | 48 | NA | 50 |
| $SiO_2/KOH$ | NA | 372 | ∞ | ∞ | 19 | ∞ |
| Surface area (m²/g) | NA | 401 | 321 | 345 | 218 | 517 |

Example 15

$H_3BO_3$ (3.54 g) was dissolved in $H_2O$ (100 g) and added to colloidal silica (172 g Ludox LS—30% $SiO_2$). To this mixture, a KOH solution (10 g, pellets—85% KOH in 100 g $H_2O$), was added. The gel was dispersed in a blender to assure adequate mixing. The reaction mixture was charged to an autoclave and crystallized at 150°C for 120 hrs while stirring under autogenous pressure. The product was filtered, washed with $Al(NO_3)_3$ (800 mls, 0.05 M), and air-dried for subsequent characterization. The major X-ray diffraction lines were at 2.0 nm, assigned as the basal spacing, and 0.34 nm which was assigned to an in-plane reflection. Other diffraction lines were present at 0.9 nm, 0.8 nm, 0.7 nm, 0.3 nm and 0.32 nm. The product has a $SiO_2/Al_2O_3$=56, $SiO_2/KOH$=1850, surface area of 146 m²/g and a value of 1.3. [27]Al nmr and temperature programmed ammonia desorption (TPAD) indicates that the Al which is detected is tetrahedrally coordinated but not all of the Al is detected. This is attributed to the Al being in a highly distorted environment.

This layered product was pillared as in the previous example to give a molecular sieve with a basal spacing of 3.67 nm, a surface area of 589 m²/g and the $SiO_2/Al_2O_3$=100. The TPAD quantitatively agrees with the bulk aluminum analysis and indicates that the aluminum is tetrahedral, zeolitic type aluminum.

Examples 16—19

The preparation of the [Al]kenyaite described in Example 15 was repeated and divided into four parts. Each part was treated with an aqueous metal salt solution in a manner similar to that described in Examples 9—13 above to either replace the Al or synthesize a mixed metal system. Table 4 lists the elemental compositions and the surface areas of the final products. The X-ray diffraction patterns of these [M]kenyaites are similar to that observed for the as-synthesized [Al]kenyaite.

These samples were pillared as in the previous examples and the properties of the pillared products are included in Table 4. The crystallinity of these molecular sieves were much better than those for Examples 9—13.

TABLE 4

| | As-syn | Ex. 16 Fe | Ex. 17 Cr | Ex. 18 Ti | Ex. 19 La |
|---|---|---|---|---|---|
| Layered phases: | | | | | |
| $SiO_2/M$ | — | 95 | 370 | 91 | 1800 |
| $SiO_2/Al_2O_3$ | 100 | 105 | 88 | 244 | 66 |
| $SiO_2/KOH$ | 40 | 3800 | 4000 | 3200 | 1200 |
| Surface area (m²/g) | NA | 137 | 147 | 162 | 143 |
| Pillared phase | | | | | |
| $SiO_2/M$ | — | 170 | 1000 | 97 | NA |
| $SiO_2/Al_2O_3$ | NA | 235 | 147 | 450 | 138 |
| $Si_2/KOH$ | NA | ∞ | ∞ | ∞ | ∞ |
| Surace area (m²/g) | NA | 574 | 551 | 569 | 528 |
| Basal spacing (nm) | NA | 3.04 | 2.76 | 2.9[a] | 2.60 |

Examples 20—27

Kenyaites were prepared the same way as in Example 14 except instead of washing the as-synthesized material with $Al(NO_3)_3$, they were washed directly with metal salt solutions (500 mls, 0.05 M). Compositions of the products are listed in Table 5.

TABLE 5

| | As-sy | Ex. 20 Fe | Ex. 21 Cr | Ex. 22 Ti | Ex. 23 La | Ex. 24 NH$_4\pm$ | Ex. 25 No wash | Ex. 26 Ga | Ex. 27 Co |
|---|---|---|---|---|---|---|---|---|---|
| Layered phase: | | | | | | | | | |
| SiO$_2$/M | 20 | 36 | 190 | 36 | 30 | 25 | — | 28 | 19 |
| SiO$_2$B$_2$O$_3$ | 115 | 132 | 132 | 145 | 425 | 150 | 8 | — | — |
| SiO$_2$/KOH | 21 | $\infty$ | $\infty$ | $\infty$ | 476 | 1200 | 17 | $\infty$ | $\infty$ |
| SA (m$^2$/g) | — | 163 | 135 | 154 | 116 | 139 | 12 | 126 | 124 |

Pillaring could then proceed as in the previous examples.

Examples 28 and 29

[Ga]kenyaite and [Fe]kenyaite were each synthesized directly by adding metal salt solution to the reaction mixture prior to crystallization. Thus, Ga(NO$_3$)$_3$ (8.5 g) or Fe(NO$_3$)$_3$ (13.5 g) was dissolved in H$_2$O and added to Ludox LS (200 g). To this solution, KOH (20 g, pellets 85%) dissolved in 100 g H$_2$O was added. The gel was aged in the steambox for about 1 hr, dispersed in 100 mls H$_2$O. The reaction mixture was crystallized at 150°C for 144 hrs under autogenous pressure in a stirred 600 ml autoclave. The product was filtered, washed with H$_2$O, and air-dried. The composition, basal spacing, and surface area of the silicate products are given in Table 6.

$^{71}$Ga nmr was used to characterize [Ga]kenyaite of Example 28. Quantitative data was not obtained but qualitatively, the Ga is tetrahedral and in a very distorted environment. No octahedrally coordinated Ga was observed.

Pillaring proceeded as before.

Examples 30 and 31

[Ga]magadiite and [Fe]magadiite were each synthesized in a manner analogous to that described in Examples 28 and 29 except that 12 g NaOH was substituted for the KOH. The composition and surface area of the products are given in Table 6. $^{71}$Ga nmr indicated again that all of the detectable Ga in the [Ga]magadiite was tetrahedral and there was no evidence of octahedral, hydrated Ga$^{3+}$(H$_2$O)$_6$ or Ga$_2$O$_3$.

TABLE 6

| | Ex. 28 Ga | Ex. 29 Fe | Ex. 30 Ga | Ex. 31 Fe |
|---|---|---|---|---|
| Layered phases: | | | | |
| SiO$_2$/M | 37 | 25 | 48 | 27 |
| SiO$_2$/XOH | 8 | 8 | NA | 360 |
| Surface area (m$^2$/g) | 5 | 23 | 46 | 42 |

**Claims**

1. A layered product comprising a non-swellable layered silicate containing non-silicon framework atoms and pillars of an oxide of at least one element selected from Groups IB, IIB, IIIA, IIIB, IVA, IVB, VA, VB, VIA, VIIA, VIIIA of the Periodic Table of the Elements separating the silicate layers.

2. The product of claim 1 wherein the silicate has the structure of kenyaite, makatite or magadiite.

3. The product of claim 1 wherein the non-silicon framework atoms are selected from Al, Zr, B, Co, Cr, Fe, Ga, In and Ni.

4. The product of claim 1 wherein the pillars are formed of a polymeric oxide.

5. The product of claim 4 wherein the pillars comprises polymeric silica.

6. A method for preparing a layered product having adjacent layers separated by pillars of an oxide of at least one element selected from Groups IB, IIB, IIIA, IIIB, IVA, IVB, VA, VB, VIA, VIIA, and VIIIA of the Periodic Table which method comprises the steps of starting with a non-swellable layered silicate which contains non-silicon framework atoms and which has anionic sites associated therewith, physically separating the layers of the silicate by introducing an organic cationic species between the layers of the anionic sites, introducing between the separated silicate layers a compound capable of conversion to an oxide and converting said compound to the chalcogenide to produce pillars of the oxide separating adjacent layers of the silicate.

7. The method of claim 6 wherein the layered silicate is produced by crystallization of an aqueous reaction mixture containing a source of silica and a source of non-silicon atom so that said atom enters the framework of the layered silicate.

8. The method of claim 7 wherein after crystallization, the non-silicon atom is at least partially replaced in the layered silicate framework with a further non-silicon atom.

9. The method of claim 6 wherein the organic cationic species is an alkylammonium cation.

10. A catalyst composite comprising the layered product of claim 1 and a matrix material.

**Patentansprüche**

1. Geschichtetes Produkt, das ein nicht quellbares geschichtetes Silicat umfaßt, das Gitteratome, die kein Silicium sind, und Stützen eines Oxids von zumindest einem Element enthält, das aus den Gruppen IB, IIB, IIIA, IIIB, IVA, IVB, VA, VB, VIA, VIIA, VIIIA des Periodensystems der Elemente ausgewählt ist, die die Silicatschichten trennen.

2. Produkt nach Anspruch 1, worin das Silicat die Struktur von Kenyait, Makatit oder Magadiit aufweist.

3. Produkt nach Anspruch 1, worin die Gitteratome, die kein Silicium sind, aus Al, Zr, B, Co, Cr, Fe, Ga, In und Ni ausgewählt sind.

4. Produkt nach Anspruch 1, worin die Stützen aus einem polymeren Oxid gebildet sind.

5. Produkt nach Anspruch 4, worin die Stützen polymeres Siliciumdioxid umfassen.

6. Verfahren zur Herstellung eines geschichteten Produktes mit benachbarten Schichten, die durch Stützen eines Oxids von zumindest einem Element getrennt werden, das aus den Gruppen IB, IIB, IIIA, IIIB, IVA, IVB, VA, VB, VIA, VIIA und VIIIA des Periodensystems ausgewählt ist, wobei dieses Verfahren die Schritte umfaßt: Beginn mit einem nicht quellbaren geschichteten Silicat, das Gitteratome enthält, die kein Silicium sind, und das damit verbundene anionische Plätze aufweist, physikalische Trennung der Schichten des Silicats durch Einführung organischer kationischer Proben zwischen die Schichten der anionischen Plätze, Einführung einer Verbindung zwischen die getrennten Silicatschichten, die in ein Oxid umwandelbar ist, und Umwandlung dieser Verbindung in das Chalkogenid, um die Stützen des Oxids zu bilden, die die benachbarten Schichten des Silicats trennen.

7. Verfahren nach Anspruch 6, worin das geschichtete Silicat durch Kristallisation einer wässrigen Reaktionsmischung hergestellt wird, die eine Siliciumdioxidquelle und eine Quelle des Atoms enthält, das kein Silicium ist, sodaß dieses Atom in das Gitter des geschichteten Silicats eintritt.

8. Verfahren nach Anspruch 7, worin das Atom, das kein Silicium ist, nach der Kristallisation im Gitter des geschichteten Silicats zumindest teilweise durch ein weiteres Atom ausgetauscht wird, das kein Silicium ist.

9. Verfahren nach Anspruch 6, worin die organischen kationischen Proben ein Alkylammoniumkation sind.

10. Katalysatorzusammensetzung, die das geschichtete Produkt von Anspruch 1 und ein Matrixmaterial umfaßt.

**Revendications**

1. Un produit feuilleté, caractérisé en ce qu'il comprend un silicate non gonflable feuilleté contenant des atomes de réseau autres que le silicium et des piliers d'un oxyde d'au moins un élément choisi parmi les Groupes IB, IIB, IIIA, IIIB, IVA, IVB, VA, VB, VIA, VIIA et VIIIA du Tableau Périodique des Eléments, séparant les couches de silicate.

2. Un produit suivant la revendication 1, caractérisé en ce que le silicate a la structure d'une kényaite, d'une makalite ou d'une magadiite.

3. Un produit suivant la revendication 1, caractérisé en ce que les atomes du réseau autres que le silicium sont choisis parmi Al, Zr, B, Co, Cr, Fe, Ga, In et Ni.

4. Un produit suivant la revendication 1, caractérisé en ce que les piliers sont formés d'un oxyde polymère.

5. Un produit suivant la revendication 4, caractérisé en ce que les piliers comprennent une silice polymère.

6. Un procédé pour préparer un produit feuilleté ayant des couches adjacentes séparées par des piliers d'un oxyde d'au moins un élément choisi parmi les Groupes IB, IIB, IIIA, IIIB, IVA, IVB, VA, VB, VIA, VIIA et VIIIA du Tableau Périodique des Eléments, caractérisé en ce qu'on par d'un silicate non gonflable feuilleté qui contient des atomes de réseau autres que le silicium et auxquels sont associés des sites anioniques, on sépare physiquement les couches du silicate en introduisant une espèce organique cationique entre les couches des sites anioniques, on introduit entre les couches séparées de silicate un composé capable de se convertir en un oxyde et l'on convertit ce composé en chalcogénure pour fournir des piliers d'oxyde séparant des couches adjacentes du silicate feuilleté.

7. Un procédé suivant la revendication 6, caractérisé en ce que le silicate feuilleté est produit par cristallisation d'un mélange réactionnel aqueux contenant une source de silice et une source d'atome autre que le silicium pour que cet atome pénètre dans le réseau du silicate feuilleté.

8. Un procédé suivant la revendication 7, caractérisé en ce qu'après la cristallisation, l'atome autre que le silicium est au moins partiellement remplacé dans le réseau du silicate feuilleté par un autre atome différent du silicium.

9. Un procédé suivant la revendication 6, caractérisé en ce que l'espèce organique cationique est un cation alkylammonium.

10. Un composite catalyseur, caractérisé en ce qu'il comprend le produit feuilleté suivant la revendication 1 et un matériau de matrice.